# EUROPEAN PATENT APPLICATION

(11) **EP 3 051 483 A1**
(43) Date of publication of application: **03.08.2016**
(21) Application number: 16153149.6
(22) Date of filing: 28.01.2016
(51) Int. Cl.: G06Q 50/00

(54) **INFORMATION PROCESSING APPARATUS, AND NON-TRANSITORY RECORDING MEDIUM**

(30) Priority: 30.01.2015 JP 2015017624; 09.12.2015 JP 2015240191
(71) Applicant: Ricoh Company, Ltd., Tokyo 143-8555 (JP)
(72) Inventor: MIZUHARA, Takuya, Tokyo, 143-8555 (JP); KASUYA, Yuuji, Tokyo, 143-8555 (JP); SUZUKI, Ryota, Tokyo, 143-8555 (JP); NAKASHIMA, Toshiharu, Tokyo, 143-8555 (JP)
(74) Representative: Schwabe - Sandmair - Marx

(57) **Abstract**

An information processing apparatus for supporting extraction, from a plurality of managed persons, of one or more persons who are subject to an interview by a manager includes an index value calculation unit configured to calculate, for each of the plurality of managed persons, an index value which is a criterion of determination whether a managed person is subject to the interview with reference to a work information database which stores work information and a health examination database which stores health examination information, a screen data generation unit configured to generate screen data for displaying a list screen, the list screen displaying the plurality of managed persons arranged in accordance with the index value and an output unit configured to output the screen data.

## Description

### BACKGROUND OF THE INVENTION

### 1. Field of the Invention

The disclosures herein generally relate to an information processing apparatus, and a non-transitory recording medium for causing an information processing apparatus to execute a process.

### 2. Description of the Related Art

In companies or various organizations (referred to as "the companies or the like" hereinafter), to perform health management while recognizing status of each employee has been required recently in terms of preventing an industrial accident or the like.

For performing the health management while recognizing the status of the employees, Japanese Unexamined Patent Application Publication No. 2003-256578 discloses a health management system which calculates a degree of risk of each of the employees becoming unhealthy physically or mentally based on a result of a health examination and a working situation. According to the health management system, the danger of impairing the health can be grasped in an early stage. The health management system displays the degree of the risk of each of the employees in a communication terminal of an industrial physician in order to cause the industrial physician to confirm whether an employee in hazardous condition exists.

In the above described health management system, in a case in which the industrial physician interviews the employees who have the risk of impairing the health, a health manager needs to confirm the degree of the risk of each of the employees. Further, considering a reason why the degree of the risk gets high and other situations, the health manager needs to determine whether the interview is necessary. Thus, the above described health management system takes time to extract (determine) the employee who is subject to the interview.

### SUMMARY OF THE INVENTION

It is a general object of at least one embodiment of the present invention to provide an information processing apparatus, and a non-transitory recording medium for causing an information processing apparatus to execute a process that substantially obviates one or more problems caused by the limitations and disadvantages of the related art.

An embodiment of the present invention provides an information processing apparatus for supporting extraction, from a plurality of managed persons, of one or more persons who are subject to an interview by a manager. The information apparatus includes an index value calculation unit configured to calculate, for each of the plurality of managed persons, an index value which is a criterion of determination whether a managed person is subject to the interview with reference to a work information database which stores work information and a health examination database which stores health examination information, the work information indicating a workplace attendance record of each of the plurality of the managed persons, the health examination information indicating a result of a health examination of each of the plurality of the managed persons, a screen data generation unit configured to generate screen data for displaying a list screen, the list screen displaying the plurality of managed persons arranged in accordance with the index value, and an output unit configured to output the screen data.

An embodiment of the present invention also provides a non-transitory recording medium for causing an information processing apparatus to execute a process, the information apparatus being for supporting extraction, from a plurality of managed persons, of one or more persons who are subject to an interview by a manager, the process including a step of calculating, for each of the plurality of managed persons, an index value which is a criterion of determination whether a managed person is subject to the interview with reference to a work information database which stores work information and a health examination database which stores health examination information, the work information indicating a workplace attendance record of each of the plurality of the managed persons, the health examination information indicating a result of a health examination of each of the plurality of the managed persons, a step of generating screen data for displaying a list screen, the list screen displaying the plurality of managed persons arranged in accordance with the index value, and a step of outputting the screen data.

### BRIEF DESCRIPTION OF THE DRAWINGS

Fig. 1 is a diagram illustrating a system configuration of a health management system according to a first embodiment;
Fig. 2 is a diagram illustrating an example of a hardware configuration of a health management server;
Fig. 3 is a diagram illustrating an example of a health examination database;
Fig. 4 is a diagram illustrating an example of a work information database;
Fig. 5 is a diagram illustrating an example of a reader database according to the first embodiment;
Fig. 6 is a diagram illustrating an example of a bad condition person discrimination model database;
Fig. 7 is a formula of the bad condition person discrimination model;
Fig. 8 is a diagram illustrating an example of a bad condition information database;
Fig. 9 is a functional block diagram of each apparatus included in the health management system according to the first embodiment;
Fig. 10 is a flowchart for describing the entire operation of the health management server according to the first embodiment;
Fig. 11 is a flowchart for describing a process of a bad condition person discrimination model learning unit according to the first embodiment;
Fig. 12 is a flowchart for describing a process of a bad condition information generation unit according to the first embodiment;
Fig. 13 is a flowchart for describing a process of a display control unit according to the first embodiment;
Fig. 14 is a first example of the list screen;
Fig. 15 is a second example of the list screen;
Fig. 16 is a third example of the list screen;
Fig. 17 is a flowchart for describing a screen transition from the list screen;
Fig. 18 is a flowchart for describing a storage process of interview necessary person information;
Fig. 19 is an example of a work information screen;
Fig. 20 is an example of a health examination information screen;
Fig. 21 is a diagram illustrating an example of an interview necessary person information screen;
Fig. 22 is a diagram illustrating a system configuration of a health management system according to a second embodiment;
Fig. 23 is a diagram illustrating an example of a reader database according to the second embodiment;
Fig. 24 is a functional block diagram of each apparatus included in the health management system according to the second embodiment;
Fig. 25 is a flowchart for describing a process of a display control unit according to the second embodiment;
Fig. 26 is a diagram illustrating a system configuration of a health management system according to a third embodiment;
Fig. 27 is a functional block diagram of each apparatus included in the health management system according to the third embodiment;
Fig. 28 is a graph illustrating a function according to the third embodiment;
Fig. 29 is a flowchart for describing a process of a bad condition information generation unit and a function generation unit;
Fig. 30 is a diagram illustrating a system configuration of a health management system according to a fourth embodiment;
Figs. 31A and 31B are formulas of a model storage unit;
Fig. 32 is a functional block diagram of each apparatus included in the health management system according to the fourth embodiment;
Fig. 33 is a flowchart for describing the entire operation of the health management server according to the fourth embodiment; and
Fig. 34 is a flowchart for describing a process of a model generation unit according to the fourth embodiment.

### DESCRIPTION OF THE PREFERRED EMBODIMENTS

In the following, embodiments of the present invention will be described with reference to the accompanying drawings.

Each of the following embodiments calculates, for each managed person managed by a health manager, an index value which is a criterion of determination whether a managed person is subject to an interview with the health manager and displays managed persons in accordance with the calculated index value in order to support extraction, from the managed persons, of one or more persons who are subject to the interview.

### (First embodiment)

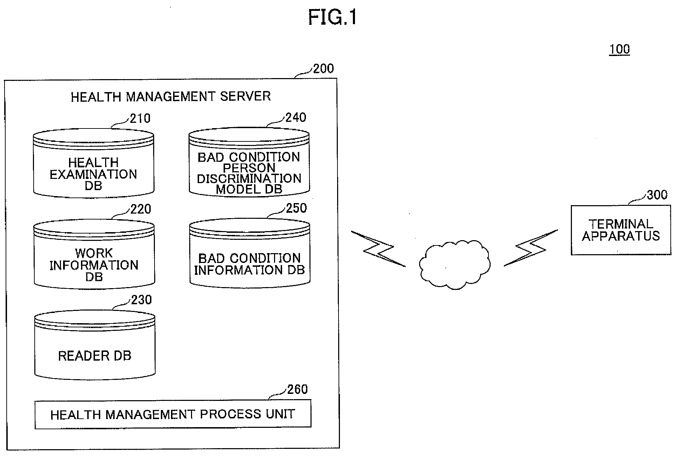

In the following, a first embodiment will be described with reference to the FIG. 1 to FIG. 21. FIG. 1 is a diagram illustrating a system configuration of a health management system 100 according to first embodiment.

The health management system 100 according to the first embodiment includes a health management server 200 and a terminal apparatus 300. The health management server 200 and the terminal apparatus 300 are connected via a communication system such as a network.

For example, the health management system 100 according to the first embodiment may be used for health management of employees by an industrial physician (doctor) or the like in the companies or the like. The industrial physician is a health manager which manages health of the employees. The employees in the companies or the like are managed persons who are managed health by the health manager.

The managed persons (managed people) according to the first embodiment are not limited to the employees in the company. The managed persons may be some persons who belong to some organization and have their health managed by the health manager. Thus, for example, the managed persons may be staff members of an organization or teachers.

The health manager according to the first embodiment is not limited to the industrial physician. For example, the health manager may be a person in a department which controls the health of the managed persons. The health manager according to the first embodiment may be a person who performs the health management of the managed persons.

Further, for example, the health management according to the first embodiment may be management performed for maintaining and enhancing the health of the managed persons. This may be performed by relieving and assuaging mental fatigue, stress, worry or the like and supporting to cope with them. Further, this may be performed by detecting and preventing mental disease, a plague or the like.

In the following descriptions, the industrial physician is described as the health manager and the employees in the company are described as the managed persons.

The health management server 200 according to the first embodiment includes a health examination database 210, a work information database 220, a reader database 230, a bad condition person discrimination model database 240, and a bad condition information database 250. Further the health management server 200 according to the first embodiment includes a health management process unit 260. The health management process unit 260 is implemented by the health management server 200 executing a health management program installed in the health management server 200.

The health management server 200 according to the first embodiment causes the health management process unit 260 to generate a bad condition person discrimination model with reference to the health examination database 210 and the work information database 220 in order to store the bad condition person discrimination model in the bad condition person discrimination model database 240. Next, the health management server 200 generates bad condition information based on the bad condition person discrimination model in order to store the bad condition information in the bad condition information database 250.

The health management server 200 calculates an index value which is a criterion of determination whether the employee is subject to an interview with the industrial physician for each of the employees using the bad condition information in order to cause the terminal apparatus 300 to display the employees in the in accordance with the calculated index value.

For example, the terminal apparatus 300 according to the first embodiment may be used by the industrial physician or the like and used for selecting the one or more persons who are subject to the interview with the industrial physician.

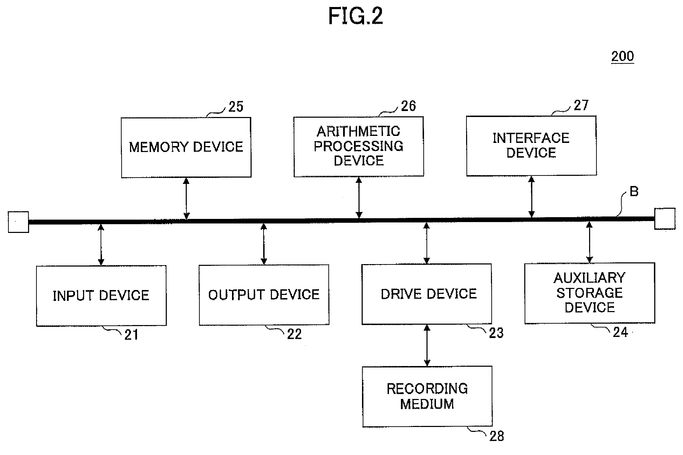

Next, a hardware configuration of the health management server according to the first embodiment will be described with reference to FIG. 2. FIG. 2 is a diagram illustrating an example of a hardware configuration of the health management server 200.

The health management server 200 according to the first embodiment is a typical information processing apparatus, and includes an input device 21, an output device 22, a drive device 23, an auxiliary storage device 24, a memory device 25, an arithmetic processing device 26, and an interface device 27 which are connected each other via a bus B.

The input device 21 may be a keyboard, a mouse, or the like, and be used for inputting various kinds of information. The output device 22 may be a display, and be used for displaying (outputting) various kinds of signals. The interface device 27 may include a modem, a local area network (LAN) card, and the like, and be used for connecting the health management server 200 to the network.

The health management program may be at least part of various kinds of programs which control the health management server 200. For example, the health management program may be provided by distributing a recording medium or downloading from the network. Various kinds of recording media such as a CD-ROM, a flexible disk, a recording medium such as a magneto optical disc which records information optically, electrically, or magnetically, a ROM, a semiconductor memory such as a flash memory which records information electrically, and the like may be used as the recording medium 28 which records the health management program.

Further, when the recording medium 28 which records the health management program is set in the drive device 23, the health management program is installed in the auxiliary storage device 24 from the recording medium 28 via the drive device 23. A communication program downloaded from the network is installed in the auxiliary storage device 24 via the interface device 27.

The auxiliary storage device 24 stores the installed health management program, necessary files, data and the like. The memory device 25 reads out and stores the health management program from the auxiliary storage device 24 when a computer starts. The arithmetic processing device 26 implements various kinds of processes, which will be described later, according to each program stored in the memory device 25.

The terminal apparatus 300 according to the first embodiment may be a typical computer. A description of a hardware configuration of the terminal apparatus 300 is omitted because the hardware configuration of the terminal apparatus 300 is similar to the hardware configuration of the health management server 200. It should be noted that in a case in which a tablet type computer or a smartphone is used as the terminal apparatus 300, the terminal apparatus 300 may include a display operation device which serves both as the input device 21 and the output device 22. For example, the display operation device may be implemented by a touch panel or the like which includes a displaying function.

Next, each database included in the health management server 200 will be described. For example, each database according to the first embodiment may be stored in the auxiliary storage device 24, the memory device 25, or the like.

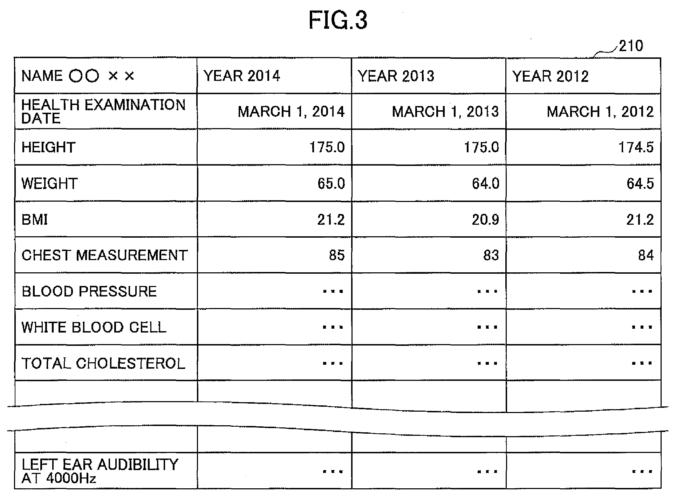

FIG. 3 is a diagram illustrating an example of the health examination database 210. The health examination database 210 according to the first embodiment is provided for each of the employees. The health examination database 210 stores results when the employees take health examinations. The health examination database 210 according to the first embodiment stores a date of the health examination, a height, a weight, a body mass index (BMI), a chest measurement, a blood pressure, a white blood cell, a total cholesterol and the like as information items. It should be noted that the health examination database 210 according to the first embodiment may include items of various kinds of inspection results performed in a typical health examination other than data the items shown in FIG. 3. In the health examination database 210 according to the first embodiment, a value of each item and the date (year, month, and day) when the health examination has been performed are stored in association with each other.

In the health examination database 210 according to the first embodiment, the value of each item is associated with the name of the employee and the date when the health examination is performed. In the following description, information which includes the name of each of the employee, the date when the health examination is performed, and the value of each item associated with the name of the employee and the date when the health examination is performed may be referred to as the health examination information. In other words, the health examination information indicates the result of the health examination of each of the employees.

As shown in FIG.3, the health examination database 210 stores the result of the of health examination on March 1, 2014, the result of the health examination on March 1, 2013, and the result of the of health examination on March 1, 2012, of the employee whose name is "○○ ××".

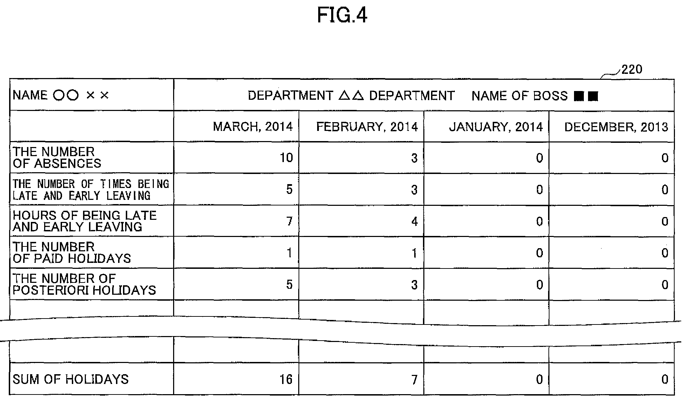

FIG. 4 is a diagram illustrating an example of the work information database 220. The work information database 220 according to the first embodiment is provided for each of the employees. The work information database 220 stores work information which indicates a workplace attendance record (working situation) of each of the employees. The work information database 220 according to the first embodiment stores the number of absences, the number of times being late and early leaving, the number of yearly paid holidays, the number of posteriori holidays and the like as information items. It should be noted that the work information database 220 according to the first embodiment may include an item of information used for typical attendance management other than the items shown in FIG. 4. In the work information database 220 according to the first embodiment, the value of each item may be, for example, stored in association with each unit (a corresponding unit) for calculating the work information. It should be noted that the number of absences indicates the number of days which the employee takes off without requesting paid holidays. It should be noted that the number of yearly paid holidays indicates the number of days which the employee takes off by requesting the holidays in advance. It should be noted that the number of posteriori holidays indicates the number of days which the employee takes off by requesting the holidays after taking off the days.

In the work information database 220 according to the first embodiment, the value of each item is associated with the name of the employee and the date when the work information is tabulated. In the following description, information which includes the name of the employee, the date when the work information is tabulated, and the value of each item associated with the name of the employee and the date when the work information is tabulated may be referred to as the work information.

As shown in FIG.4, the work information is tabulated for every month. The work information database 220 stores the work information on December 2013, the work information on January 2014, the work information on February 2014, and the work information on March 2014, of the employee whose name is "○○ ××".

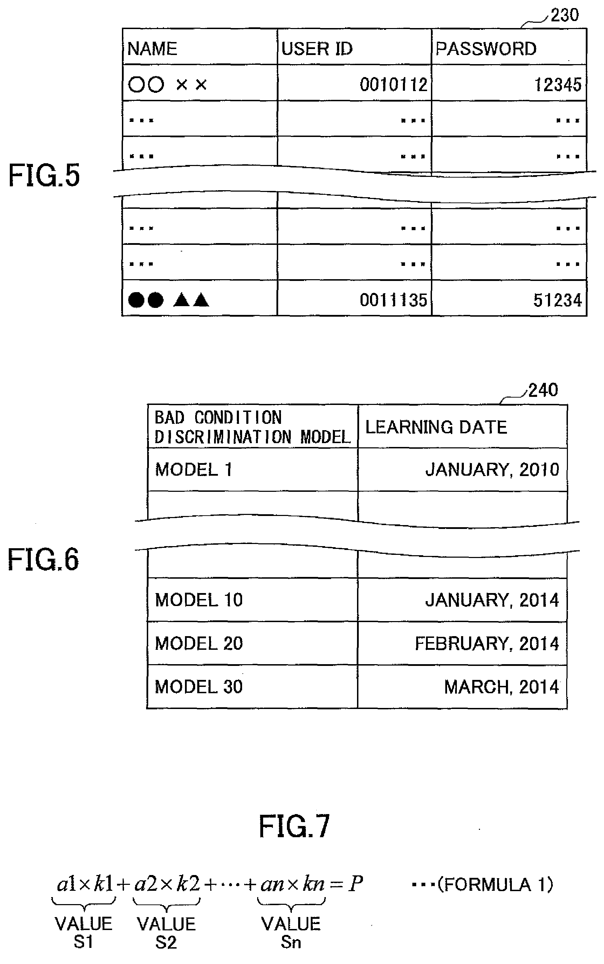

FIG. 5 is a diagram illustrating an example of the reader database 230 according to the first embodiment. For example, the reader (viewer) according to the first embodiment may be the health manager such as the industrial physician.

The reader database 230 according to the first embodiment stores identification information of the health manager. The reader database 230 according to the first embodiment stores a name, a user ID, a password as information items. The value of the item "NAME" may be the name of the industrial physician. The value of the item "user ID" may be identification information given to the industrial physician. The value of the item "PASSWORD" may be a password set for each industrial physician.

In the first embodiment, for example, the industrial physician at the terminal apparatus 300 may input the user ID and the password in the terminal apparatus 300 in order to login to the health management system 100 when reading (viewing) the information included in the health management server 200.

FIG. 6 is a diagram illustrating an example of the bad condition person discrimination model database 240. The bad condition person discrimination model database 240 according to the first embodiment stores a bad condition person discrimination model and a date of learning as information items.

The value of the item "BAD CONDITION PERSON DISCRIMINATION MODEL" is a bad condition person discrimination model which will be described later. The value of the item "LEARNING DATE" indicates a date when the bad condition person discrimination model is learned (generated).

As shown in FIG. 6, the bad condition person discrimination model database 240 indicates that a bad condition person discrimination model 10 is generated on January 2014, a bad condition person discrimination model 20 is generated on February 2014, and a bad condition person discrimination model 30 is generated on March 2014.

In the following, the bad condition person discrimination model according to the first embodiment will be described with reference to FIG. 7. FIG. 7 is a formula of the bad condition person discrimination model.

The bad condition person discrimination model according to the first embodiment may be, for example, a linear discriminant function obtained by discrimination analysis as shown in FIG. 7 (formula (1)).

The discrimination analysis is a method for obtaining a standard (discrimination function) for discriminating one of groups into which newly obtained data are put, in a case in which it is apparent that data given previously are put into different groups.

The health management server 200 according to the first embodiment uses the health examination information accumulated in the health examination database 210 for each of the employees and the work information accumulated in the work information database 220 for each of the employees as data given previously, and generates the bad condition person discrimination model shown in formula (1) by a known method.

The bad condition person discrimination model according to the first embodiment is the sum of a first value and a second value. The first value is obtained by multiplying the value of each item included in the work information database 220 by a coefficient calculated for (determined with respect to) each item. The second value is obtained by multiplying the value of each item included in the health examination database 210 by a coefficient calculated (determined with respect to) for each item.

For example, in FIG. 7, "a1" may be the value of the item "THE NUMBER OF ABSENCES" in the work information database 220 and the value of the item "k1" may be a constant number which corresponds to "THE NUMBER OF ABSENCES". Further, "a2" may be the value of the item "THE NUMBER OF TIMES BEING LATE AND EARLY LEAVING" in the work information database 220 and the value of item "k2" may be a constant number which corresponds to "THE NUMBER OF TIMES BEING LATE AND EARLY LEAVING".

As described above, the first embodiment generates the bad condition person discrimination model which includes the value of each item in the work information database 220, the constant number (coefficient) which corresponds to the value of each item in the work information database 220, the value of each item in the health examination database 210, and the constant number (coefficient) which corresponds to the value of each item in the health examination database 210.

In the following description, the value P which is obtained as the output of the bad condition person discrimination model is referred to as the bad condition score. The bad condition score is used for calculating, for each of the employees, the index value which is the criterion of determination whether the employee is subject to the interview with the industrial physician.

Further, in the following description, the value S1 obtained by multiplying "a1" by "k1" is referred to as an item "term of "THE NUMBER OF ABSENCES"", and the value S2 obtained by multiplying "a2" by "k2" is referred to as an item "TERM OF "THE NUMBER OF TIMES BEING LATE AND EARLY LEAVING"". Further, in the following description, the value of each term in the bad condition person discrimination model is referred to as the value of "term of item name".

In the first embodiment, the bad condition information, which will be described later, includes the bad condition score, the index value, and the value of the term of each item.

It should be noted that although the linear discriminant function is used for the bad condition person discrimination model according to the first embodiment as shown in FIG. 7, the present invention is not limited to this. For example, in the first embodiment, the bad condition person discrimination model may be generated by using an algorithm such as a neural network. The neural network is a mathematical model for expressing some characteristics which can be shown in brain function according to simulation on a computer.

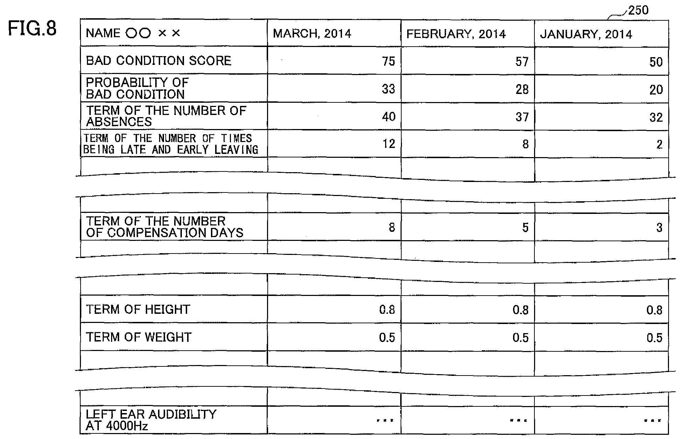

FIG. 8 is a diagram illustrating an example of the bad condition information database 250. The bad condition information database 250 according to the first embodiment is provided for each of the employees. The bad condition information database 250 according to the first embodiment stores the bad condition score, a probability of the bad condition, a value of each item included in the work information database 220, and a term of each item included in the health examination database 210, as information items.

The value of the item "BAD CONDITION SCORE" is the value P which is obtained as the result of calculation according to the bad condition person discrimination model. In the first embodiment, it may be indicated that the greater the value of the bad condition score is, the higher the possibility of the problem of the health status is. That is, the greater the value of the bad condition score is, the greater the probability of being in the bad condition is.

The value of the item "PROBABILITY OF BAD CONDITION" is the index value which is the criterion of the determination whether the employee is subject to the interview with the industrial physician. More specifically, the item "PROBABILITY OF BAD CONDITION" indicates the percentage of the employees who have taken leave from work among the employees each of who has the same value of the bad condition score. The description of the item "PROBABILITY OF BAD CONDITION" will be described later.

Further, the item "term of each item" has been described in FIG. 7. For example, the value of the item "TERM OF "THE NUMBER OF ABSENCES"" is S1, and the value of the item "TERM OF "THE NUMBER OF TIMES BEING LATE AND EARLY LEAVING"" is S2.

In the bad condition information database 250 according to the first embodiment, the value of item is associated with the name of the employee and the date when the bad condition information is generated. In the following description, information which includes the name of the employee, the date when the bad condition information is generated, and the value of each item, which is associated with the name of the employee and the date when the bad condition information is generated, is referred to as the bad condition information.

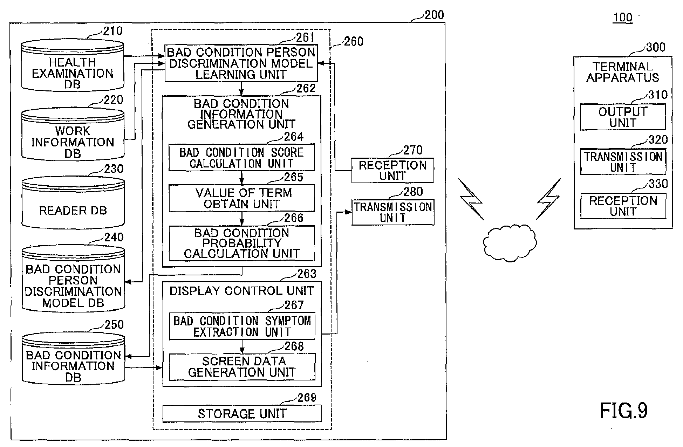

Next, a function of the health management server 200 according to the first embodiment will be described with reference to FIG. 9. FIG. 9 is a functional block diagram of each apparatus included in the health management system 100 according to the first embodiment.

The health management server 200 according to the first embodiment includes a health management process unit 260, a transmission unit 280, and a reception unit 270.

The health management process unit 260 according to the first embodiment is implemented by the arithmetic processing device 26 executing the health management program.

The transmission unit 280 according to the first embodiment transmits data to the terminal apparatus 300 and/or other external terminals. The reception unit 270 receives the data from the terminal apparatus 300 and/or the other external terminals.

The health management process unit 260 according to the first embodiment includes a bad condition person discrimination model learning unit 261, a bad condition information generation unit 262, a display control unit 263, and a storage unit 269.

In response to an event that the reception 270 receives, from the terminal apparatus 300, an instruction to start an extraction process of the person who is subject to the interview, the bad condition person discrimination model learning unit 261 generates the bad condition person discrimination model with reference to the health examination database 210 and the work information database 220. The bad condition information generation unit 262 generates the bad condition information using the bad condition person discrimination model. The display control unit 263 generates screen data of a screen displayed by the terminal apparatus 300 using the generated bad condition information. The storage unit 269 stores a part of the bad condition information of the employee who is selected as the person who is subject to the interview.

The bad condition information generation unit 262 according to the first embodiment includes a bad condition score calculation unit 264, a value of term obtain unit 265, and a bad condition probability calculation unit 266.

The bad condition score calculation unit 264 calculates the bad condition information score based on the bad condition person discrimination model. The value of term obtain unit 265 obtains the value of each term which is included in the bad condition person discrimination model and obtained in the process of calculating the bad condition score. The bad condition probability calculation unit 266 calculates the probability of the bad condition based on the bad condition score and the work information.

The display control unit 263 according to the first embodiment includes a bad condition symptom extraction unit 267, and a screen data generation unit 268.

The bad condition symptom extraction unit 267 extracts the item which corresponds to the term whose value obtained by the term of value obtain unit 265 is equal to or greater than a predetermined threshold. In other words, the bad condition symptom extraction unit 267 extracts, from each item of the work information database 220 and the health examination database 210, one or more items in which the value of the term, which is the multiplied value obtained by multiplying the value of each item and the coefficient determined with respect to each item, is equal to or greater than the predetermined threshold. Specifically, for example, in a case in which the value S1 of the term is equal to or greater (more) than the predetermined threshold, the bad condition symptom extraction unit 267 may extract the item "THE NUMBER OF ABSENCES" which corresponds to the value S1 of the term and the value of the item "THE NUMBER OF ABSENCES" in the work information database 220.

The screen data generation unit 268 generates screen data which includes the probability of the bad condition, the item extracted by the bad condition symptom extraction unit 267, and the value of the item.

The generated screen data is transmitted to the terminal apparatus 300 via the transmission unit 280.

The storage unit 269 stores the name of the employee selected in the terminal apparatus 300 and the item which is extracted by the bad condition symptom extraction unit 267 from the bad condition information as information of the person who needs to take the interview (the interview necessary person information).

Next, a function of the terminal apparatus 300 will be described. The terminal apparatus 300 according to the first embodiment includes an output unit 310, a transmission unit 320, and a reception unit 330.

For example, the output unit 310 may output the screen data which is received by the reception unit 330 from the health management server 200 to a display or the like in order to display the screen data. For example, the transmission unit 320 may transmit, from the terminal apparatus 300, the instruction to start the extraction process of the person who is subject to the interview to the health management server 200. For example, the reception unit 330 may receive the screen data displayed by the terminal apparatus 300 from the health management server 200.

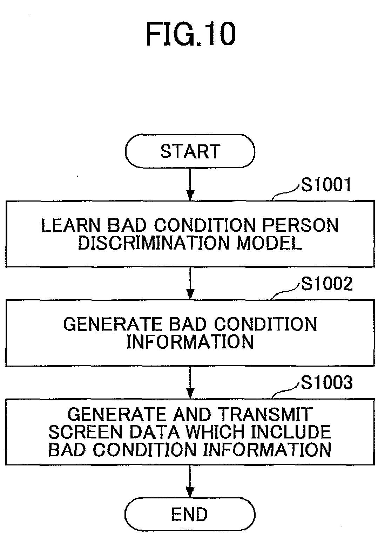

Next, an operation of the health management server 200 according to the first embodiment will be described. FIG. 10 is a flowchart for describing the entire operation of the health management server 200 according to the first embodiment.

In response to receiving, from the terminal apparatus 300, the instruction to start the extraction process of the person who is subject to the interview, the health management server 200 according to the first embodiment causes the bad condition person discrimination model learning unit 261 to generate the bad condition person discrimination model in step S1001.

Subsequently, the health management server 200 causes the bad condition information generation unit 262 to perform the calculation of the generated bad condition person discrimination model with reference to the health examination database 210 and the work information database 220 in order to generate the bad condition information in step S1002.

Subsequently, the health management server 200 generates the screen data which includes the part of the bad condition information in order to transmit the screen data to the terminal apparatus 300 in step S1003.

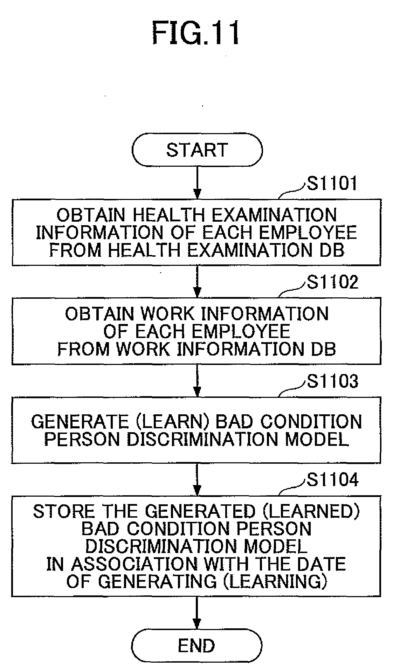

In the following, the process in steps S1001 to S1003 shown in FIG. 10 will be described in detail. FIG. 11 is a flowchart for describing a process of the bad condition person discrimination model learning unit 261. FIG. 11 shows the details of the process in step S1001 shown in FIG. 10.

In the health management server 200 according to the first embodiment, in response to receiving, from the terminal apparatus 300, the instruction to start the extraction process of the person who is subject to the interview, the bad condition person discrimination model learning unit 261 obtains the health examination information of each of the employees with reference to the health examination database 210 in step S1101.

It should be noted that when receiving the instruction to start the extraction process of the person who is subject to the interview, the health management server 200 may cause the terminal apparatus 300 to display a screen for inputting the user ID and the password. Then, in a case in which the user ID and the password, which are input, are stored in the reader database 230 of the health management server 200, the process goes to a step which will be described later. In a case in which the user ID and the password, which are input, are not stored in the reader database 230, the health management server 200 may cause the terminal apparatus 300 to display an error message.

Subsequently, the bad condition person discrimination model learning unit 261 obtains the work information of each of the employees with reference to the work information database 220 in step S1102.

Subsequently, the bad condition person discrimination model learning unit 261 generates the bad condition person discrimination model using the health examination information and the work information in step S1103. Subsequently, the bad condition person discrimination model learning unit 261 stores the generated bad condition person discrimination model in the bad condition person discrimination model database 240 in association with the information which indicates the date when the bad condition person discrimination model is generated in step S1104.

As described above, the first embodiment generates a new bad condition person discrimination model from the latest health examination information and the latest work information every time receiving, from the terminal apparatus 300, the instruction to start the extraction process of the person who is subject to the interview. Thus, the first embodiment can update the bad condition person discrimination model every time the health examination information and the work information are accumulated. In the first embodiment, as described above, to generate the bad condition person discrimination model from the information which includes the latest health examination information and the latest work information is referred to as "to learn the bad condition person discrimination model".

It should be noted that although the bad condition person discrimination model is generated in response to receiving, from the terminal apparatus 300, the instruction to start the extraction process of the person who is subject to the interview as shown in FIG. 11, the present invention is not limited to this. For example, the bad condition person discrimination model learning unit 261 according to the first embodiment may generate the bad condition person discrimination model in a case in which one of the health examination database 210 and the work information database 220 is updated.

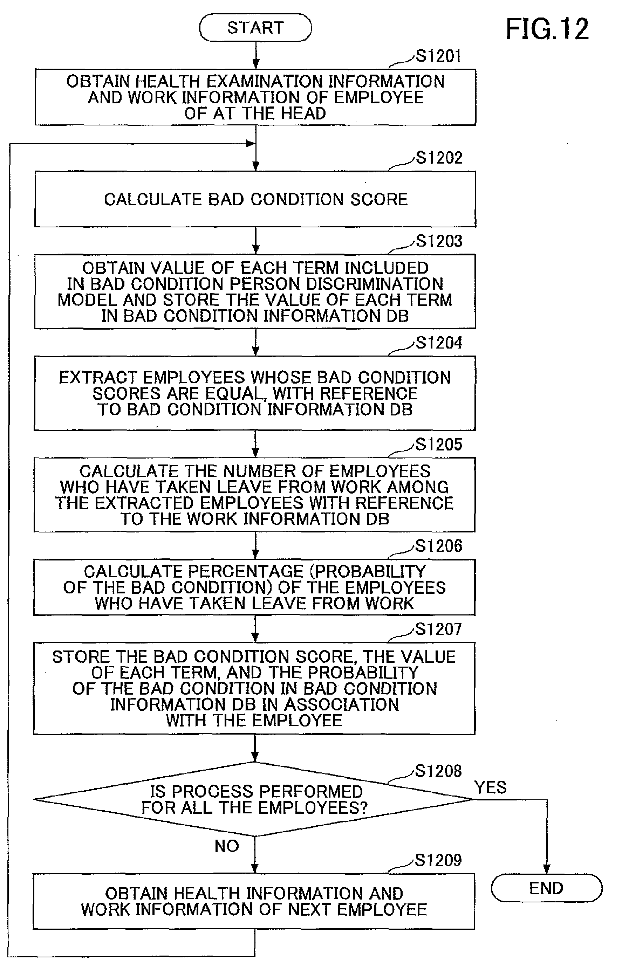

FIG. 12 is a flowchart for describing a process of the bad condition information generation unit 262. FIG. 12 shows the detail of the process in step S1002 shown in FIG. 10.

The bad condition information generation unit 262 according to the first embodiment obtains a name of an employee of at the head of the health examination database 210 and corresponding examination information. Further, the bad condition information generation unit 262 obtains, from the work information database 220, the work information which corresponds to the obtained name in step S1201.

Subsequently, using the value of each item of the health examination information and the work information obtained in step S1201, the bad condition information generation unit 262 causes the bad condition score calculation unit 264 to perform the calculation of the bad condition person discrimination model in order to calculate the bad condition score in step S1202. For example, in the first embodiment, in a case in which a linear discriminant analysis is used for the bad condition person discrimination model as learning algorithm, the output of the linear discriminant function is the bad condition score. Further, in the first embodiment, in a case in which the neural network is used for the bad condition person discrimination model, the output signal of the output layer is the bad condition score.

Further, the bad condition score calculation unit 264 according to the first embodiment may calculate the bad condition score using the latest bad condition person discrimination model stored in the bad condition person discrimination model database 240. Further, the bad condition score calculation unit 264 according to the first embodiment may use the bad condition person discrimination model generated at a time similar to a time when at least one of the work information and the health examination information is updated. For example, in a case in which both of the work information and the health examination information are updated on March 2014, the bad condition score calculation unit 264 may use the bad condition person discrimination model generated on March 2014.

Subsequently, the bad condition information generation unit 262 causes the value of term obtain unit 265 to store, in the bad condition information database 250, the item which correspond to each term included in the bad condition person discrimination model and the value of the term in association with each other in step S1203.

Subsequently, the bad condition information generation unit 262 causes the bad condition probability calculation unit 266 to extract the name of the employee whose bad condition score is equal to the bad condition score calculated in step S1202 with reference to the bad condition information database 250 in step S1204. Subsequently, the bad condition probability calculation unit 266, with reference to the work information database 220, calculates the number of employees who have taken leave from work or who are taking leave from work in step S1205 among the employees whose names are extracted in step S1204.

Subsequently, the bad condition probability calculation unit 266 calculates the percentage of the employees who have taken leave from work among the employees whose names are extracted in step S1204, and defines the calculation result as the probability of the bad condition in step S1206. That is, the first embodiment defines the percentage of the employees who have taken leave from work among the employees whose bad condition scores are equal to the employee who is subject to the generation of the bad condition information as the probability of the bad condition. In other words, the first embodiment calculates the percentage of the employees each of who has a history of taking the leave from work among the employees each of who has a bad condition score which matches a bad condition score of the employee who is a calculation target of the index value with reference to the work information database 210 and the health examination database 220.

Specifically, for example, in a case in which the bad condition score calculated in step S1202 is 20, the number of employees whose bad condition score are 20 is 10, and the number of employees who have taken leave from work is 8, the probability of the bad condition is 80%.

Subsequently, the bad condition information generation unit 262 stores the bad condition score, the value of each term, and the probability of the bad condition in the bad condition information database 250 as the bad condition information in association with the name of the employee (identification information for identifying the employee) in step S1207. At that time, the bad condition information generation unit 262 also stores information which indicates the date when the bad condition information is generated in association with the bad condition information.

Next, the bad condition information generation unit 262 determines whether the bad condition information is generated for all the employees in step S1208. In a case in which the bad condition information generation unit 262 determines that the bad condition information is not generated for all the employees (NO in step S1208), the bad condition information generation unit 262 obtains the health information and the work information of next employee in step S1209, and the process goes to step S1202.

In a case in which the bad condition information generation unit 262 determines that the bad condition information is generated for all the employees (YES in step S1208), the bad condition information generation unit 262 finishes the process.

It should be noted that although the name of the employee is used as information for identifying the employee in the description of FIG. 12, the present invention is not limited to this. The process in FIG. 12, an employee number given to each of the employees may be used for the information for identifying the employee.

Here, the probability of the bad condition according to the first embodiment will be described. In the first embodiment, the probability of the bad condition is the percentage of the employees who have taken leave from work among the employees each of who has the same bad condition score.

There is a tendency that the company is required to perform the health management in mentality because the number of employees who take leave from work because of the bad condition in the mentality has been increasing recently. In the first embodiment, the employee who has high probability of the bad condition indicates that the number of employees who have taken leave from work is large among the employees each of who has the same value of the bad condition score. That is the employee who has high probability of the bad condition indicates that the possibility that the employee will take leave from work is high.

According to the first embodiment, to use the value of the probability of the bad condition which indicates the possibility of taking leave from work as the index value which is the criterion of the determination whether the employee is subject to the interview, makes it easy for the industrial physician to determine whether the employee is in the bad condition in a mental aspect. In other words, the index value indicates the probability of leave from work to be taken by the employee.

It should be noted that although the first embodiment emphasizes the importance of detecting the bad condition of the mentality in an early stage and defines the value which indicates the possibility of taking leave from work as the probability of the bad condition, the present invention is not limited to this. For example, a value which corresponds to another item included in the health examination information may be defined as the probability of the bad condition, and a value which corresponds to an item other than the item which corresponds to leave from work included in the work information may be defined as the probability of the bad condition.

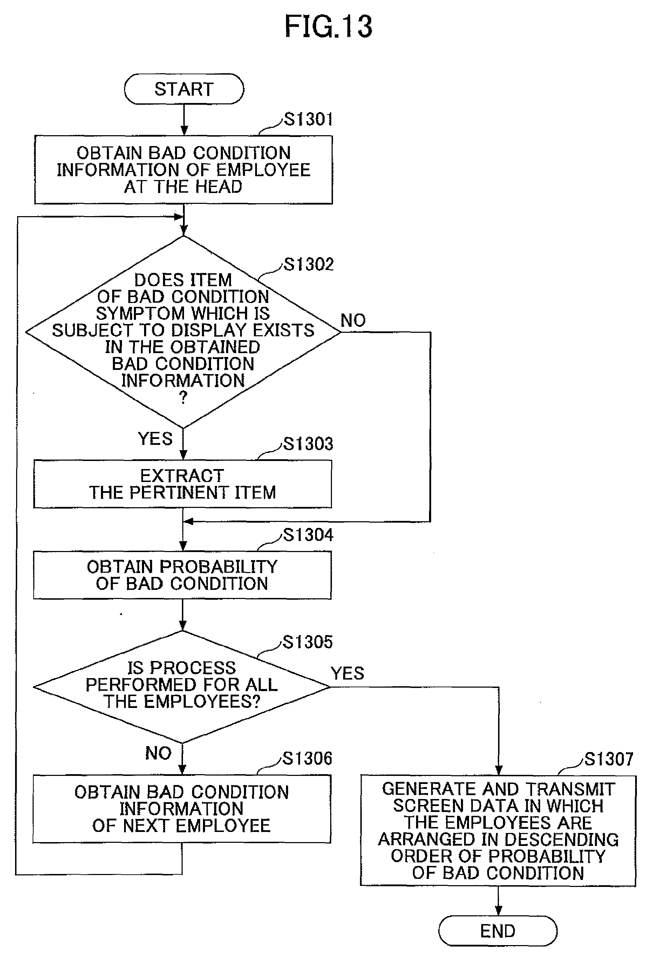

Next, a process of the display control unit 263 according to the first embodiment will be described. FIG. 13 is a flowchart for describing a process of the display control unit 263 according to the first embodiment.

The display control unit 263 according to the first embodiment obtains the bad condition information of the employee at the head of the bad condition information database 250 in step S1301. Subsequently, the display control unit 263 causes the bad condition symptom extraction unit 267 to determine whether an item of a bad condition symptom which is subject to display exists in the bad condition information in step S1302.

In the following, the determination in step S1302 will be described. The bad condition symptom extraction unit 267 according to the first embodiment extracts the term whose value is greater than the predetermined threshold with reference to the items and the value of each item in the work information database 220 and the items and the value of each item in the health examination database 210 which are included in the bad condition information. Then, the bad condition symptom extraction unit 267 defines the item which corresponds to the extracted term as the bad condition symptom item.

The term whose value is greater than the predetermined threshold is a term which has high contribution to the bad condition score.

For example, in a case in which the value S1 of the item "TERM OF THE "THE NUMBER OF ABSENCES"" and the value S2 of the item "TERM OF THE "THE NUMBER OF TIMES BEING LATE AND EARLY LEAVING"" are greater than the predetermined threshold, the item "THE NUMBER OF ABSENCES" and the item "THE NUMBER OF TIMES BEING LATE AND EARLY LEAVING", which correspond to each term, become the bad condition symptom items (refer to FIG. 7). It should be noted that the predetermined threshold for determining whether the item is the bad condition symptom item is previously set. For example, the predetermined threshold may be set by the manager of the health management server 200, or by the industrial physician from the terminal apparatus 300.

In a case in which the bad condition symptom exists (YES in step S1302), the bad condition symptom extraction unit 267 extracts the pertinent item (one or more items) from the health examination database 210 and/or the work information database 220 in step S1303. In a case in which the bad condition symptom does not exist (NO in step S1302), the process goes to step S1304 which will be described later.

Subsequently, the display control unit 263 obtains, from the bad condition information, the probability of the bad condition which corresponds to the employee at the head in step S1304.

Subsequently, the display control unit 263 determines whether the process in step S1302 to step S1304 is performed for all the employees in step S1305. In a case in which the process in step S1302 to step S1304 is not performed for all the employees (NO in step S1305), the display control unit 263 obtains the bad condition information of the next employee in step S1306. Then the process returns to step S1302.

In a case in which the process in step S1302 to step S1304 is performed for all the employees (YES in step S1305), the display control unit 263 causes the screen data generation unit 268 to generate screen data of a screen in which the employees are arranged in the descending order of the probability of the bad condition, and causes the transmission unit 280 to transmit the screen data to the terminal apparatus 300 in step S1307.

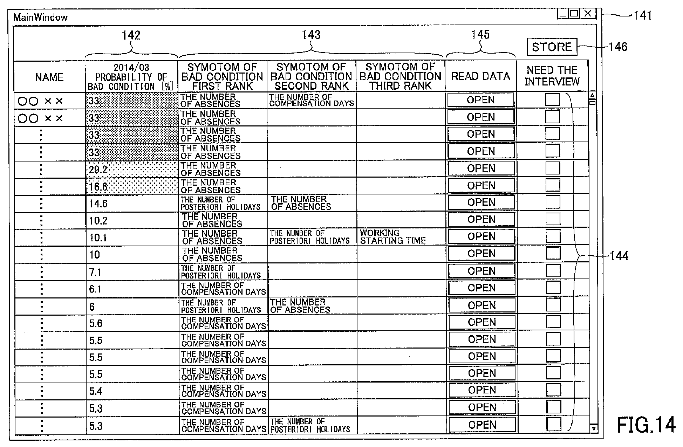

In the following, a list screen will be described with reference to FIG. 14.

FIG. 14 is a first example of the list screen. A list screen 141 shown in FIG. 14 is an example of a screen displayed in the terminal apparatus 300.

The list screen 141 includes bad condition probability fields 142, bad condition symptom item fields 143, selection fields 144, detail reading buttons 145, and a storage button 146.

The probability of the bad condition of each of the employees is displayed in the respective bad condition probability fields 142. The items of the bad condition symptom of each of the employees are displayed in the respective bad condition symptom item fields 143. The selection fields 144 are fields for selecting one or more employees as the persons who are subject to the interview. The detail reading buttons 145 are buttons for transitioning the list screen 141 to a screen in which the work information and/or the health examination information of the corresponding employee is displayed. The storage button 146 is a button for storing the item of the bad condition symptom of the employee selected as the person who is subject to the interview as the information of the person who needs to take the interview (the interview necessary person information).

In the list screen 141 according to the first embodiment, for example, a marker may be added to a predetermined number of persons in the bad condition probability fields or an emphasized character may be used.

Further, although, three items of the bad condition symptom are displayed in the bad condition symptom item fields 143 according to the first embodiment, the number of the items of the bad condition symptom being displayed is not limited to this. The number of the items of the bad condition symptom displayed in the list screen 141 may be an arbitrary number.

The selection fields 144 according to the first embodiment are provided for each of the employees. In the example shown in FIG. 14, when one of the selection fields 144, which are boxes, is selected, a check is displayed in the box and it can be seen that the corresponding employee is selected. It should be noted that the selection fields 144 shown in FIG. 14 are the example, and the present invention is not limited to this. It is preferable that the selection fields 144 according to the first embodiment are configured such that the employee selected as the person who is subject to the interview can be seen in the list screen 141.

In response to selecting the detail reading buttons 145 according to the first embodiment, the list screen 141 is changed to one of a work information screen and a health examination information screen. The work information screen displays the work information of the employee who corresponds to the selected detail reading button 145. The health examination information screen displays the health examination information of the employee who corresponds to the selected detail reading button 145.

In response to operating the storage button 146 according to the first embodiment, information which includes the probability of the bad condition and the item of the bad condition symptom of the employee selected in the list screen 141 as the person who is subject to the interview is stored.

In the example shown in FIG. 14, the probability of the bad condition of the employee whose name is "○○ ××* is 33%, and the items of the bad condition symptom are "THE NUMBER OF ABSENCES" and "THE NUMBER OF COMPENSATION DAYS". FIG. 14 shows that among the values of the terms of the bad condition information of this employee, the value of the term which corresponds to the item "THE NUMBER OF ABSENCES" is the largest and the value of the term which corresponds to the item "THE NUMBER OF COMPENSATION DAYS" is the second largest.

Further, as shown in FIG. 14, third item of the bad condition symptom which corresponds to the employee whose name is "○○ ××" is a blank. This indicates that the bad condition information of the employee whose name is "○○ ××" has no items whose values of the term are equal to or greater than the predetermined threshold except for the item "THE NUMBER OF ABSENCES" and the item "THE NUMBER OF COMPENSATION DAYS". That is, the list screen 141 indicates that the possibility that the employee whose name is "○○ ××" has the problem of the health status is high, mainly in terms of the number of absences and the number of compensation days.

Similarly, the employee whose name is "BB bb" has no items whose value of the term are equal to or greater than the predetermined threshold except for the item "THE NUMBER OF ABSENCES". The possibility that the employee whose name is "BB bb" has the problem of the health status is high, mainly in terms of the number of absences.

Further, in response to selecting the detail reading button 145 which corresponds to the employee whose name is "○○ ××" in FIG. 14, the health management server 200 causes the display control unit 263 to display the work information and the health examination of the employee whose name is "○○ ××".

When the selection field 144 which corresponds to the employee whose name is "○○ xx" is selected and the storage button 146 is operated in FIG. 14, the health management server 200 stores the interview necessary person information in which the name "○○ ××" of the employee and the items of the bad condition symptom are associated with each other.

It should be noted that although the employees are arranged in the descending order of the probability of the bad condition, and the name of the employee, the probability of the bad condition, and the items of the bad condition symptom are displayed in the list screen 141 according to the first embodiment in association with each other, the present invention is not limited to this.

For example, in the list screen 141, the employees may be arranged in the ascending order of the probability of the bad condition, and the name of the employee, the probability of the bad condition, and the items of the bad condition symptom may be displayed in association with each other.

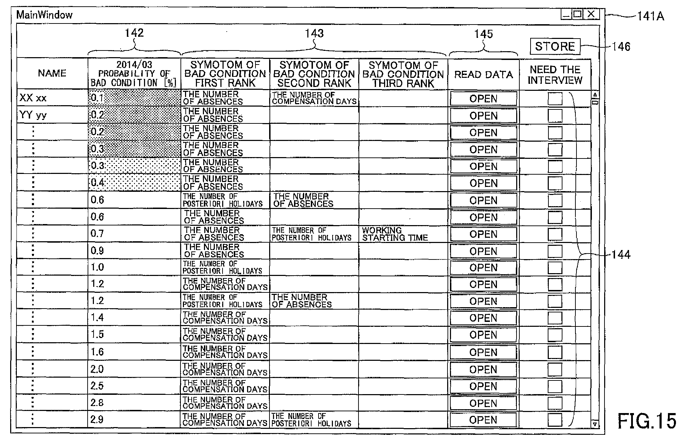

FIG. 15 is a second example of the list screen. In the example shown in FIG. 15, the employees are arranged in the ascending order of the probability of the bad condition, and the name of the employee, the probability of the bad condition, and the items of bad condition symptom are displayed in association with each other.

To display the employees as shown in FIG. 15 may enable to see (read) a total transition of the probability of the bad condition of each of the employees when the industrial physician selects the person who is subject to the interview, for example.

Further, in the above described embodiment, highness of the probability of taking leave from work is referred to as highness of the probability of the bad condition. The wording "the probability of the bad condition" is an example for describing the index value which is the criterion of the determination whether the employee is subject to the interview. Thus, instead of the wording "the probability of the bad condition", another wording may be used.

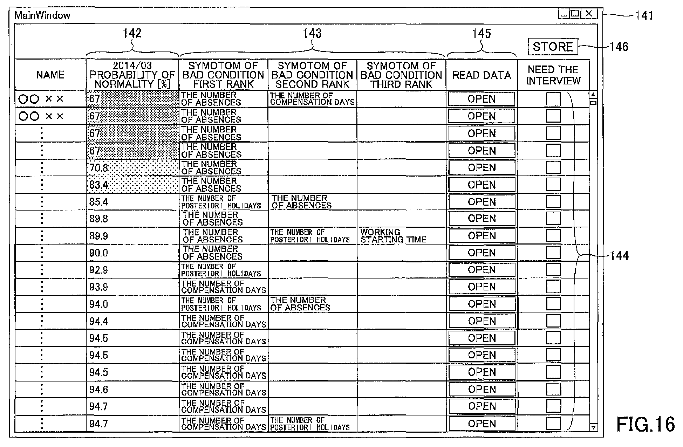

FIG. 16 is a third example of the list screen. In the example shown in FIG. 16, instead of the wording "PROBABILITY OF BAD CONDITION", a wording "PROBABILITY OF NORMALITY" is used.

The probability of the normality (normal condition) indicates that the more the value of the probability of the normality is, the less the probability of taking leave from work is, and the less the value of the probability of the normality is, the more the probability of taking leave from work is. In other words, the employee, whose probability of the normality is high, has low probability of taking leave from work, and the employee, whose probability of the normality is low, has high probability of taking leave from work.

The probability of the normality may be a value, for example, obtained by subtracting the probability of the bad condition from 100%. In the example shown in FIG. 16, the employees are arranged in the ascending order of the probability of the normality, and the name of the employee, the probability of the bad condition, and the items of the bad condition symptom are displayed in association with each other. Thus, in the example shown in FIG. 16, the bad condition information is displayed in the descending order of the probability of taking leave from work.

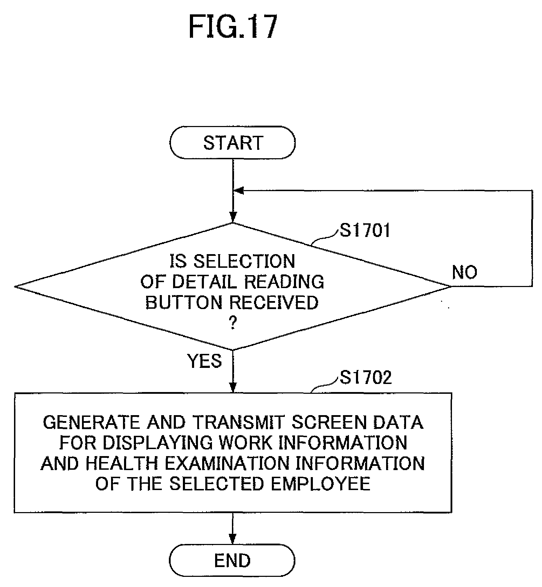

Next, screen transition from the list screen 141 will be described with reference to FIG. 17. FIG. 17 is a flowchart for describing the screen transition from the list screen 141. FIG. 17 shows a process of the display control unit 263 in a case in which the detail reading button 145 is selected in the list screen 141.

The health management server 200 according to the first embodiment causes the display control unit 263 to determine whether a selection of the detail reading buttons 145 is received in the list screen 141 in step S1701. That is, the display control unit 263 determines whether a display request of details of the work information and the health examination information of the employee which corresponds to the selected detail reading button 145 is received.

In a case in which the display request is received (YES in step S1701), the display control unit 263 obtains the work information of the pertinent employee from the work information database 220 and the health examination information of the pertinent employee from the health examination database 210. Then, the display control unit 263 causes the screen data generation unit 268 to generate the screen data which includes the work information screen and the health examination information screen, causes the transmission unit 280 to transmit the screen data to the terminal apparatus 300 in step S1702, and finish the process.

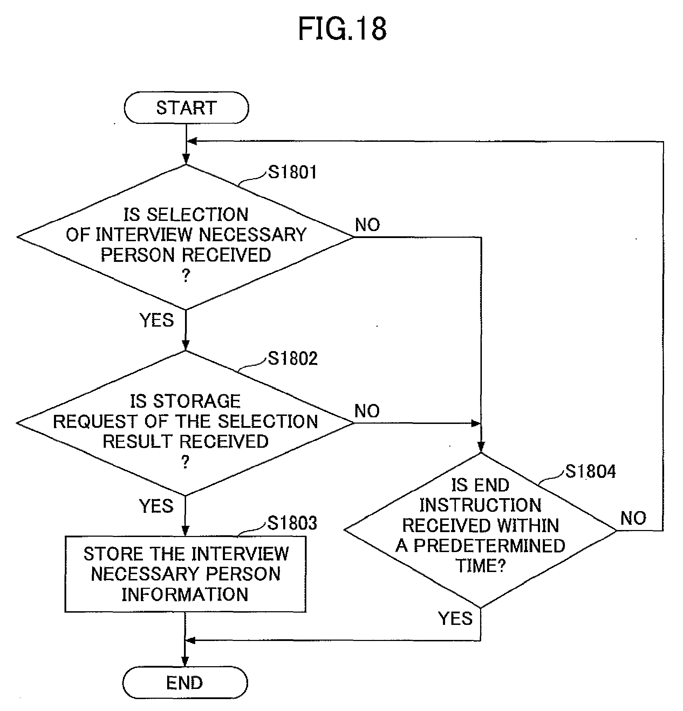

Next, a storage process of the interview necessary person information by the storage unit 269 will be described with reference to FIG. 18. FIG. 18 is a flowchart for describing the storage process of the interview necessary person information. FIG. 18 shows a process of a case in which the storage button 146 is selected after the selection fields 144 are selected in the list screen 141.

The display control unit 263 according to the first embodiment determines whether a selection of the interview necessary person is received in the list screen 141 in step S1801. In a case in which the selection is not received (NO in step S1801), the process goes to step S1804 which will be described later.

In a case in which the selection is received (YES in step S1801), the display control unit 263 determines whether a storage request of the selection result is received in step S1802. Specifically, the display control unit 263 determines whether the storage button 146 is selected after the employee is selected in the list screen 141.

In a case in which the storage button 146 is selected (YES in step S1802), the storage unit 269 stores the interview necessary person information in which the name of the selected employee (the interview necessary person) and the items of the bad condition symptom are associated in step S1803. Then, the process ends. For example, the storage unit 269 according to the first embodiment may store the interview necessary person information as a file form which can be displayed with a predetermined application.

In the case in which the interview necessary person is not received (NO in step S1801) and in the case in which the storage request of the result of the selection is not received (NO in step S1802), the display control unit 263 determines whether an end instruction of displaying is received within a predetermined time in step S1804. In a case in which the end instruction is not received within the predetermined time (NO in step S1804), the process returns to step S1801 and waits. In a case in which the end instruction is received within the predetermined time (YES in step S1804), the display control unit 263 finish the process.

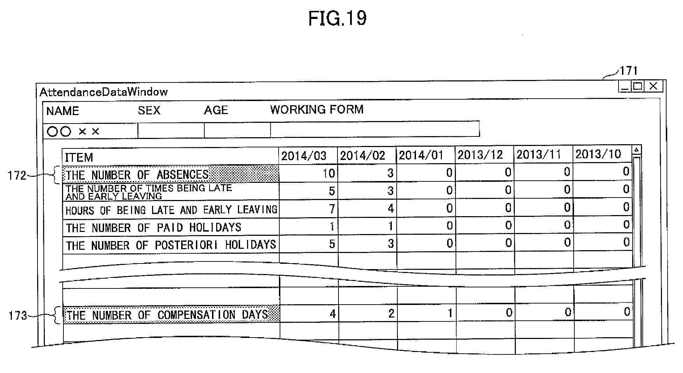

Next, the work information screen according to the first embodiment will be described with reference to FIG. 19. FIG. 19 is an example of the work information screen.

The work information of the employee whose name is "○○ ××" is displayed in the work information screen 171 as shown in FIG. 19.

In a case in which the item of the bad condition symptom, extracted from the work information, exists, the display control unit 263 according to the first embodiment may add a marker to the extracted item when displaying the work information screen 171.

The items of the bad condition symptom of the employee whose name is "○○ ××" are the item "THE NUMBER OF ABSENCES" and the item "THE NUMBER OF COMPENSATION DAYS".
Thus, in the work information screen 171 shown in FIG. 19, the marker is added to a display field 172 of the item "THE NUMBER OF ABSENCES" and a display field 173 of the item "THE NUMBER OF COMPENSATION DAYS".

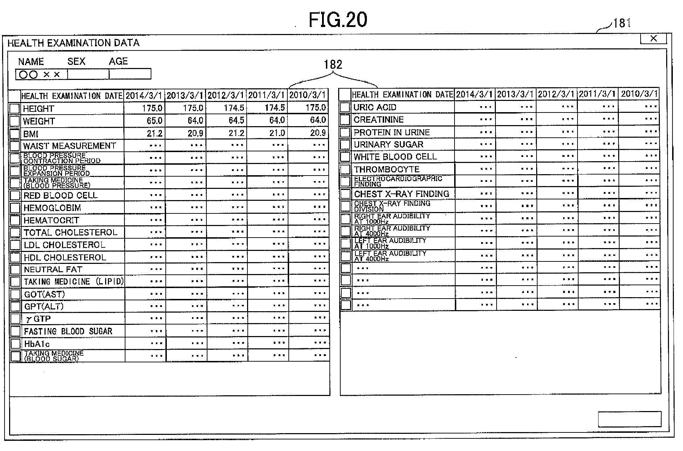

Next, the health examination information screen according to the first embodiment will be described with reference to FIG. 20. FIG. 20 is an example of the health examination information screen 181.

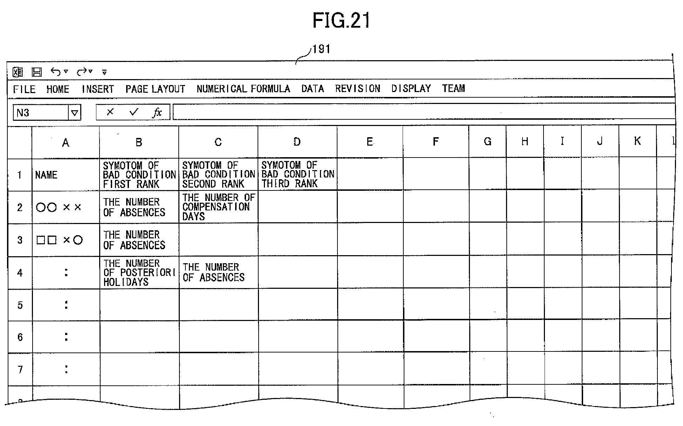

The health examination information screen 181 shown in FIG. 21 displays the health examination information of the employee whose name is "○○ ××".

Lists 182 of the health examination information are displayed in the health examination information screen 181. Although the lists 182 are divided into two rows as shown in FIG. 20, the present invention is not limited to this. The lists 182 may be one row in which the items and the value of each item are associated, and seen by scrolling.

Next, the interview necessary person information will be described with reference to FIG. 21. FIG. 21 is a diagram illustrating an example of the interview necessary person information screen 191.

The interview necessary person information screen 191 shown in FIG. 21 displays an example of the interview necessary person information in a case in which employees including the employee whose name is "○○ ××" and the employee whose name is "□□ ×○" are selected as the interview necessary persons in the list screen 141.

In the interview necessary person information screen 191 according to the first embodiment, the name "○○ ××", the item "THE NUMBER OF ABSENCES" as the item of the bad condition symptom, and the item "THE NUMBER OF COMPENSATION DAYS" as the item of the bad condition symptom are associated with each other. Further, the name "□□ xo", the item "THE NUMBER OF ABSENCES" as the item of the bad condition symptom are associated with each other.

It should be noted that although the interview necessary person information according to the first embodiment includes (displays) only the name and the items of the bad condition symptom, the present invention is not limited to this. For example, the interview necessary person information may include the bad condition score, the probability of the bad condition or the like other than the name and the items of the bad condition symptom.

As described above, using the work information and the health examination information of each of the employees, the first embodiment calculates the index value which is the criterion of the determination whether the employee is subject to the interview, and displays the list screen 141 in which the employees (managed persons) are arranged in accordance with the index value. Thus, according to the first embodiment, the health manager may easily estimate (select) the one or more managed persons who are required to take the interview. Further, the first embodiment may support the efficient extraction of the one or more persons who are subject to the interview.

### (Second embodiment)

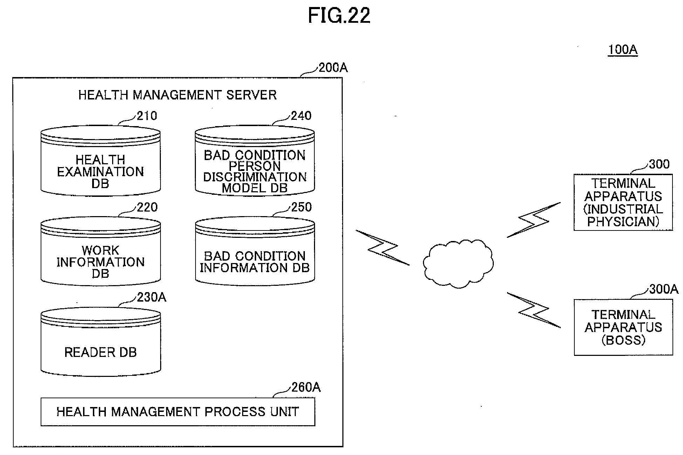

In the following, a second embodiment will be described with reference to FIG. 22 to FIG. 25. The second embodiment differs from the first embodiment in that, according to the second embodiment, information displayed by the terminal apparatus 300 is selected in accordance with the occupation of the health manager who reads the information. In the following description of the second embodiment, differences between the second embodiment and the first embodiment will be described. The same reference numbers are given to functional configurations of the second embodiment similar to the functional configurations of the first embodiment, and their descriptions may be omitted as appropriate.

FIG. 22 is a diagram illustrating a system configuration of a health management system 100A according to the second embodiment. The health management system 100A according to the second embodiment includes a health management server 200A and terminal apparatuses 300 and 300A. The health management server 200A and the terminal apparatuses 300 and 300A are connected via the communication system such as the network.

The health management server 200A according to the second embodiment includes a reader database 230A instead of the reader database 230. Further, the health management server 200A includes a health management process unit 260A.

The terminal apparatus 300A according to the second embodiment may have the same configuration as the terminal apparatus 300. For example, the terminal apparatus 300A may be used by a boss of the employee or the like. In the following description, in a case in which the terminal apparatus 300 and the terminal apparatus 300A are not distinguished, an arbitrary terminal apparatus is referred to as the terminal apparatus 300.

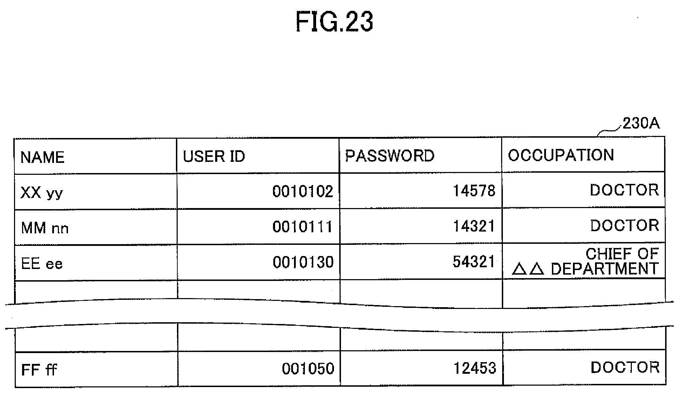

FIG. 23 is a diagram illustrating an example of the reader database 230A according to the second embodiment. The reader database 230A according to the second embodiment stores identification information of the health manager and information which indicates an occupation of the health manager in association with each other. The reader database 230A according to the second embodiment includes a name, a user ID, a password, and an occupation as items of information. A value of the item "OCCUPATION" indicates the occupation of the reader. It should be noted that the value of the item "OCCUPATION" may include information of department of the reader. In the example shown in FIG. 23, the occupation of the employee whose name is "XX yy" is doctor, the occupation of the employee whose name is "EE ee" is chief of ΔΔ department.

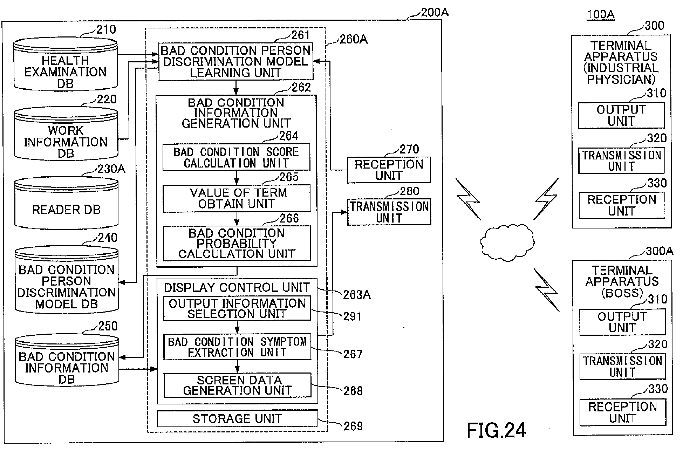

FIG. 24 is a functional block diagram of the apparatuses included in the health management system 100A according to the second embodiment.

The health management server 200A according to the second embodiment includes a health management process unit 260A. The health management process unit 260A according to the second embodiment is implemented by the arithmetic processing device 26 executing the health management program.

The health management process unit 260A according to the second embodiment includes a display control unit 263A. The display control unit 263A includes an output information selection unit 291 in addition to the units included in the display control unit 263 according to the first embodiment.

The output information selection unit 291 according to the second embodiment selects information to be displayed in accordance with the occupation of the reader of the terminal apparatus 300 when the reception unit 270 receives, from the terminal apparatus 300, a display request of the screen list in which the employees are arranged in the descending order of the probability of the bad condition, a display request of the work information, or a display request of the health examination information.

Specifically, in a case in which the occupation of the reader is the doctor, in response to the request of the reader (doctor), the output information selection unit 291 causes the terminal apparatus 300 to display the requested information from all the information sources including the health examination information. Further, in a case in which the occupation of the reader is an occupation except the doctor, in response to the request of the reader (except the doctor), the output information selection unit 291 causes the terminal apparatus 300 to display the requested information from the information except the health examination information.

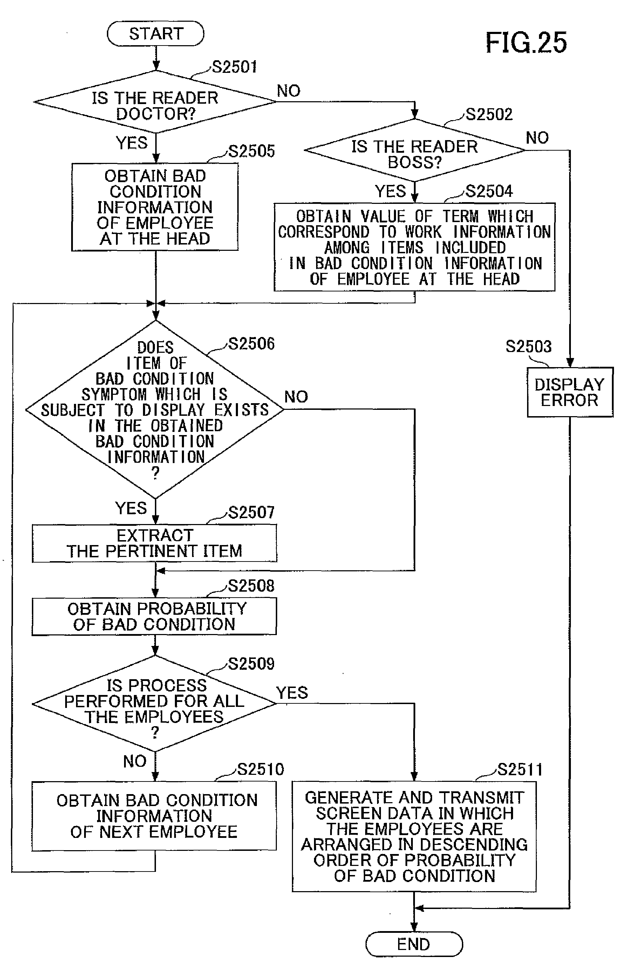

FIG. 25 is a flowchart for describing a process of the display control unit 263A according to the second embodiment. In response to receiving, from the terminal apparatus 300, a start instruction of the extraction process of the person who is subject to the interview, the display control unit 263A according to the second embodiment causes the terminal apparatus 300 to display a screen for inputting the user ID and the password. Then, the display control unit 263A determines whether the occupation which corresponds to the user ID which is input is the doctor with reference to the reader database 230A in step S2501.

In a case in which the occupation which corresponds to the user ID is not the doctor (NO in step S2501), the display control unit 263A determines whether the occupation which corresponds to the user ID is a managerial position (boss) in step S2502. In a case in which the occupation which corresponds to the user ID is not the managerial position (NO in step S2502), the display control unit 263A causes the terminal apparatus 300 to display an error message in step S2503. Then, the process ends. The error message may indicate that the user is not allowed to see the information.

In a case in which the occupation which corresponds to the user ID is the managerial position (YES in step S2502), the display control unit 263A obtains the value of the term which corresponds to the items included in the work information database 220 among the bad condition information of the employee at the head of the bad condition information database 250 in step S2504, and the process goes to step S2506.

In a case in which the occupation which corresponds to the user ID is the doctor (YES in step S2501), the process goes to step S2505 which will be described later.

Because processes in step S2505 to step S2511 shown in FIG. 25 are similar to the processes in step S1301 to step S1307 shown in FIG. 13, their descriptions will be omitted.

As described above, the second embodiment may enable to read (inspect) the bad condition information which includes the health examination information only when the reader is the doctor. The second embodiment may prohibit (restricts) reading (inspecting) the health examination information when the reader is in the managerial position. As described above, the second embodiment may protect personal information with regard to health statuses of the employees who are the managed persons by selecting the information which is displayed by the terminal apparatus 300.

It should be noted that the second embodiment may allow only users whose official positions are higher than a predetermined official position among users whose occupations are the managerial positions to read the work information of the employees. Further, the second embodiment may allow a user whose occupation is the managerial position to read only the work information of employees who belong to the own department managed by the user.

Further, in the display control unit 263A according to the second embodiment, the screen data generation unit 268 may generate screen data of a screen for displaying information selected by the output information selection unit 291 when the detail reading buttons 145 are selected in the list screen 141, for example. That is, in the case in which the occupation of the reader is the doctor, both the work information screen 171 and the health examination information screen 181 can be displayed in the terminal apparatus 300. Further, in the case in which the occupation of the reader is the managerial position, only the work information screen 171 can be displayed in the terminal apparatus 300.

It should be noted that although the second embodiment selects the information displayed by the terminal apparatus 300 in accordance with the occupation of the reader, the present invention is not limited to this.

For example, the output information selection unit 291 may select information which includes the items of the bad condition symptom from the health examination information and the work information and display the information which includes the item of the bad condition symptom. Specifically, for example, in a case in which the item of the bad condition symptom is the item "THE NUMBER OF ABSENCES", the output information selection unit 291 may display only the work information of the pertinent employee.

### (Third embodiment)

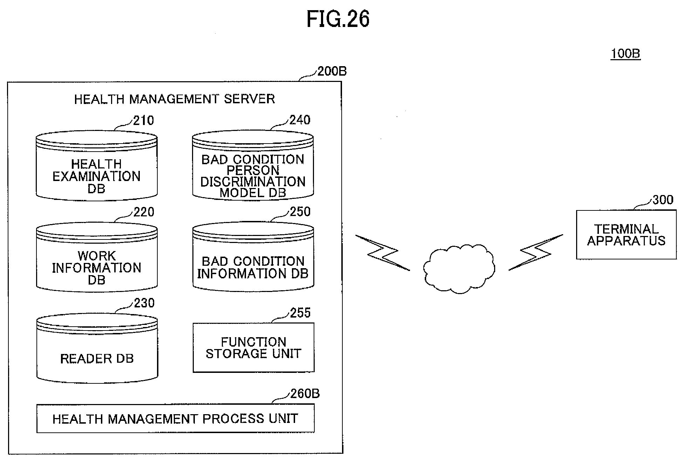

In the following, a third embodiment will be described with reference to FIG. 26 to FIG. 29. The third embodiment differs from the first embodiment in that the third embodiment generates a function for calculating the probability of the bad condition based on the bad condition score. In the following description of the third embodiment, differences between the third embodiment and the first embodiment will be described. The same reference numbers are given to functional configurations of the third embodiment similar to the functional configurations of the first embodiment, and their descriptions may be omitted as appropriate.

FIG. 26 is a diagram illustrating a system configuration of a health management system 100B according to the third embodiment. The health management system 100B according to the third embodiment includes a health management server 200B and the terminal apparatus 300.

The health management server 200B according to the third embodiment includes a function storage unit 255 in addition to the databases included in the first embodiment. Further, the health management server 200B includes a health management process unit 260B.

When the bad condition information is generated, the health management process unit 260B according to the third embodiment generates a function which indicates a relationship between the bad condition score and the probability of the bad condition (the index value) based on the generated bad condition information, and stores the function in the function storage unit 255. And, when a process for calculating the probability of the bad condition has newly occurred, the health management process unit 260B calculates the probability of the bad condition based on the bad condition score and the generated function.

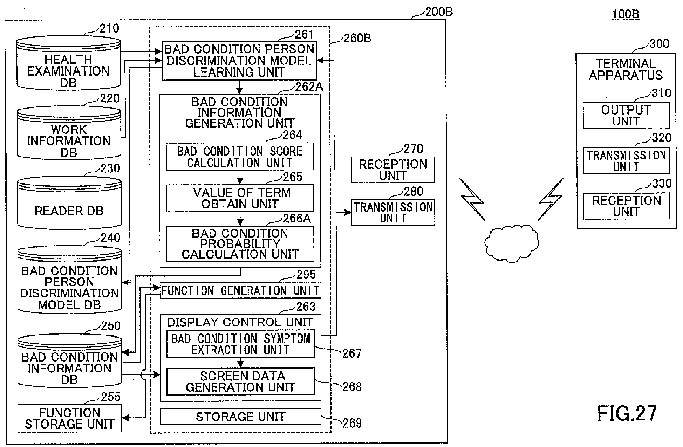

FIG. 27 is a functional block diagram of the apparatuses included in the health management system 100B according to the third embodiment.

The health management process unit 260B included in the health management server 200B according to the third embodiment includes a bad condition person discrimination model learning unit 261, a bad condition information generation unit 262A, the display control unit 263, and a function generation unit 295.

The bad condition information generation unit 262A according to the third embodiment includes a bad condition probability calculation unit 266A. The bad condition probability calculation unit 266A according to the third embodiment calculates the probability of the bad condition in a different way from the bad condition probability calculation unit 266 according to the first embodiment. Specifically, the bad condition probability calculation unit 266A according to the third embodiment calculates the number of employees who have taken leave from work among the employees each of whose bad condition scores is equal to or greater than X as a probability of the bad condition of an employee whose bad condition score is X. A process of the bad condition probability calculation unit 266A will be described later.

The function generation unit 295 according to the third embodiment generates a bad condition probability calculation function which indicates the relationship between the bad condition score and the probability of the bad condition based on the bad condition score and the bad condition information of each of the employees generated by the bad condition information generation unit 262A.

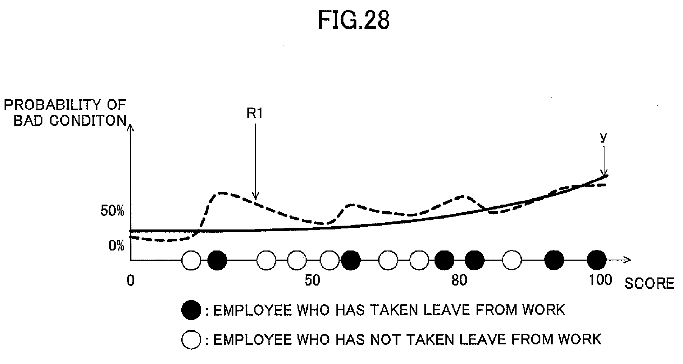

In the following, the function which indicates the relationship between the bad condition score and the probability of the bad condition will be described with reference to FIG. 28. FIG. 28 is a graph illustrating the function according to the third embodiment.

In FIG. 28, the abscissa indicates the bad condition score and the ordinate indicates the probability of the bad condition. Black circles on the abscissa indicate employees, whose bad condition scores are indicated by the black circles, who have taken leave from work. White circles indicate employees, whose bad condition scores are indicated by the white circles, who have not taken leave from work.

A curve line R1 shown in FIG. 28 is drawn by plotting the probability of the bad condition calculated by the bad condition probability calculation unit 266A with respect to the bad condition score.

In the third embodiment, for example, when calculating the probability of the bad condition of the employee whose bad condition score is X, the bad condition probability calculation unit 266A extracts the employees each of whose bad condition scores is equal to or greater than X, and calculates a percentage of employees who have taken the leave from work among the employees each of whose bad condition scores is equal to or greater than X as the probability of the bad condition.

For example, a case of an employee whose bad condition score is 80 will be described with reference to FIG. 28.
In this case, among the employees whose bad condition scores are equal to or greater than 80, the number of employees who have taken the leave from work is three and the number of employees who have not taken the leave from work is one. Thus, the probability of the bad condition before calculating a function y is 75% (3/4).

Thus, according to the third embodiment, for example, in a case in which the number of the employees whose bad condition score are 80 is one, the probability of the bad condition of the employee can be calculated properly.

It should be noted that although the third embodiment calculates the percentage of the number of employees who have taken the leave from work among the number of the employees each of whose bad condition scores is equal to or greater X as the probability of the bad condition which corresponds to the bad condition score X, the present invention is not limited to this. For example, the probability of the bad condition may be a percentage of the number of employees who have taken the leave from work among the number of employees whose bad condition scores are within a predetermined range. In this case, a center value in the predetermined range may be the bad condition score X. Further, some embodiment may extract employees whose bad condition scores are less than X and employees whose bad condition scores are greater than X by a predetermined number of persons in order to calculate a percentage of the number of employees who have taken the leave from work among the number of extracted employees as the probability of the bad condition. In other words, the first embodiment calculates, with reference to the work information database 220 and the health examination database 210, a percentage of employees each of who has a history of taking the leave from work in employees each of whose bad condition scores is within the predetermined range, the predetermined range including a bad condition score of the employee who is a calculation target of the index value.

For example, in FIG. 28, the probability of the bad condition of the four employees whose bad condition score is around 80 is 50% because the number of employees who have taken leave from work is two and the number of employees who have not taken leave from work is two.

Further, the third embodiment may calculate the function y which indicates the relationship between the bad condition score and the probability of the bad condition by fitting (setting) the bad condition score and the probability of the bad condition to a quadratic function. When fitting, the third embodiment may use, for example, a least squares method.

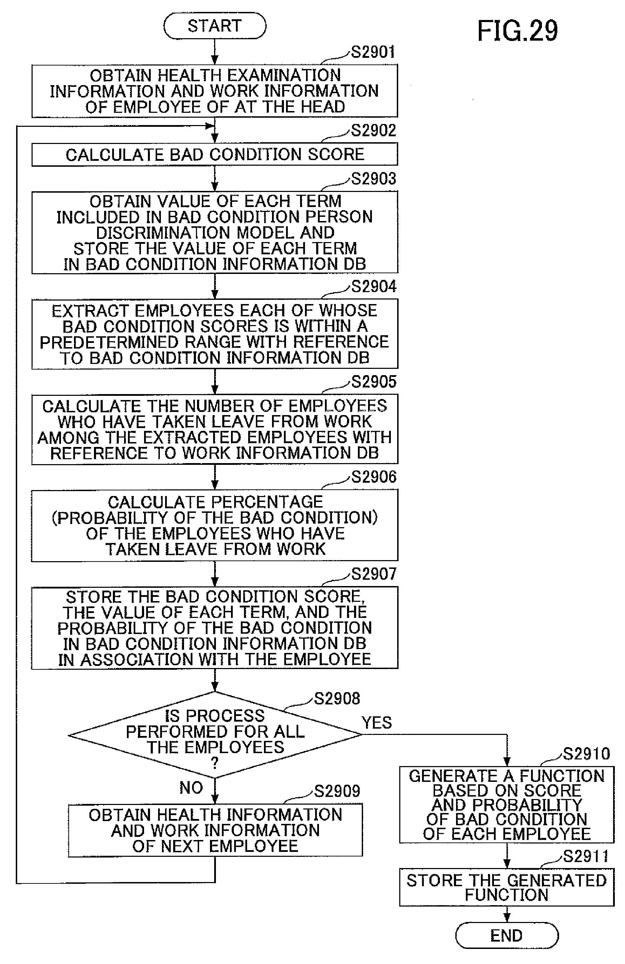

Next, a process of the bad condition information generation unit 262A and the function generation unit 295 will be described with reference to FIG. 29.

FIG. 29 is a flowchart for describing the process of the bad condition information generation unit 262A and the function generation unit 295.

Because processes in step S2901 to step S2903 shown in FIG. 29 are similar to the processes in step S1201 to step S1203 shown in FIG. 12, their descriptions will be omitted.

Subsequent to step S2903, in step S2904, the bad condition information generation unit 262A causes the bad condition probability calculation unit 266A to extract, from the bad condition information database 250, employees each of whose bad condition scores is within a predetermined range. The predetermined range includes the bad condition score calculated in step S2902.

For example, the predetermined range may be a range from the calculated bad condition score to 100 which is the upper limit of the bad condition score. Further, the predetermined range may be a range whose center value is the calculated bad condition score.

Because processes in step S2905 to step S2909 shown in FIG. 29 are similar to the processes in step S1205 to step S1209 shown in FIG. 12, their descriptions will be omitted.

In a case in which a process for each of the employees is finished (Yes in step S2908), the function generation unit 295 generates the function y based on the bad condition score and the probability of the bad condition of each of the employees with reference to the bad condition information database 250 in step S2910.

Subsequently, the function generation unit 295 stores the generated function y in the function storage unit 255 in step S2911. Then, the process ends.

As described above, the third embodiment can calculate (generate) the function y which indicates the relationship between the bad condition score and the probability of the bad condition, and calculates the probability of the bad condition by substituting the bad condition score in the function y. Thus, the process for calculating the probability of the bad condition can be simplified, for example, in a case in which the bad condition information of a newly added employee is generated.

### (Fourth embodiment)

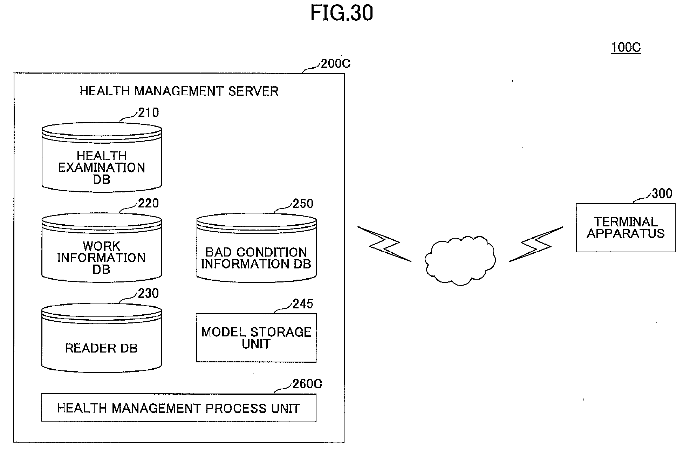

In the following, a fourth embodiment will be described with reference to FIG. 30 to FIG. 34. The fourth embodiment differs from the first embodiment in that the fourth embodiment holds the function which indicates the relationship between the bad condition score and the probability of the bad condition as a model. In the following description of the fourth embodiment, differences between the fourth embodiment and the first embodiment will be described. The same reference numbers are given to functional configurations of the fourth embodiment similar to the functional configurations of the first embodiment, and their descriptions may be omitted as appropriate.

FIG. 30 is a diagram illustrating a system configuration of a health management system 100C according to the fourth embodiment. The health management system 100C according to the fourth embodiment includes a health management server 200C and the terminal apparatus 300.

The health management server 200C according to the fourth embodiment includes a model storage unit 245 instead of the bad condition person discrimination model database 240. Further, the health management server 200C according to the fourth embodiment includes a health management process unit 260C.

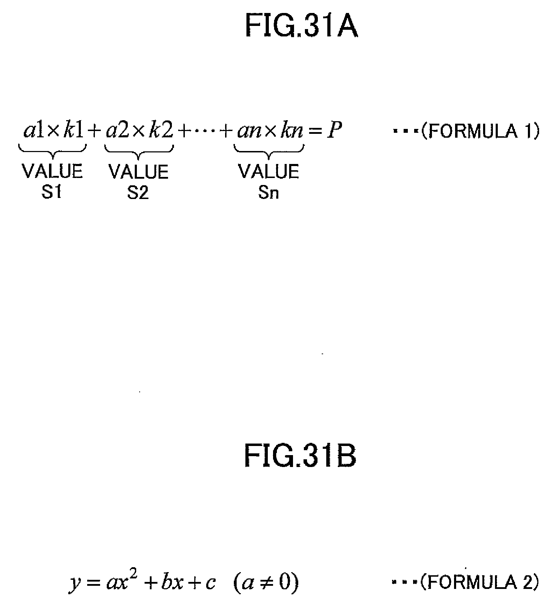

FIGs. 31A and 31B are formulas of the model storage unit 245. As shown in FIG. 31A and FIG. 31B, the model storage unit 245 according to the fourth embodiment stores the formula (1) which is the bad condition person discrimination model and the formula (2) which indicates the function y.

The fourth embodiment generates the function y when the bad condition person discrimination model is generated in order to store the function y in the model storage unit 245.

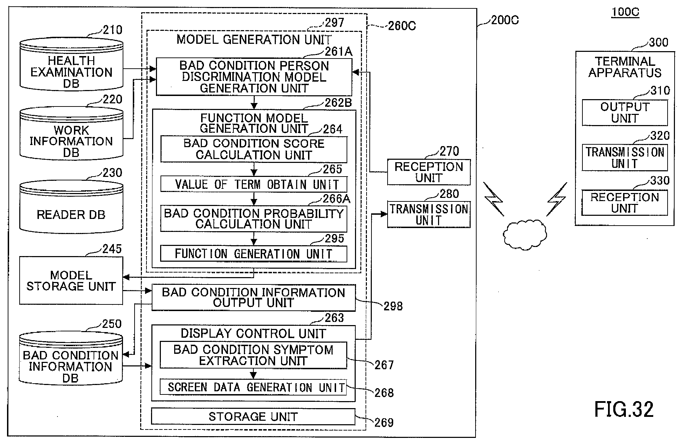

FIG. 32 is a functional block diagram of the apparatuses included in the health management system 100C according to the fourth embodiment.

The health management server 200C according to the fourth embodiment includes a health management process unit 260C. The health management process unit 260C includes a model generation unit 297, a bad condition information output unit 298, the display control unit 263, and the storage unit 269.

The model generation unit 297 according to the fourth embodiment includes a bad condition person discrimination model generation unit 261A and a function model generation unit 262B.

The bad condition person discrimination model generation unit 261A generates a bad condition person discrimination model. The bad condition person discrimination model generation unit 261A according to the fourth embodiment differs from the bad condition person discrimination model learning unit 261 according to the first embodiment only in that the bad condition person discrimination model generation unit 261A does not generates the bad condition person discrimination model in accordance with the update of the health examination database 210 or the work information database 220. A process of generation of the bad condition person discrimination model according to the fourth embodiment is similar to the first embodiment.

The function model generation unit 262B according to the fourth embodiment includes the bad condition score calculation unit 264, the value of term obtain unit 265, the bad condition probability calculation unit 266A, and the function generation unit 295. The processes of the units are similar to the processes described in the first embodiment or the third embodiment.

The bad condition information output unit 298 according to the fourth embodiment calculates and outputs the bad condition information of each of the employees based on the bad condition person discrimination model generated in the model generation unit 297 and the function y in order to store the bad condition information of each of the employees in the bad condition information database 250.

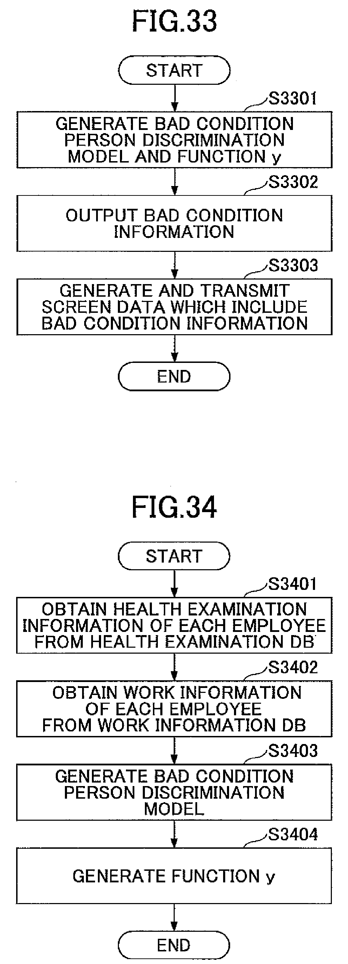

FIG. 33 is a flowchart for describing the entire operation of the health management server 200C according to the fourth embodiment.

The health management server 200C according to the fourth embodiment causes the bad condition person discrimination model generation unit 261A of the model generation unit 297 to generate the bad condition person discrimination model and causes the function model generation unit 262B to generate the function y in step S3301.

Subsequently, the health management server 200C causes the bad condition information output unit 298 to calculate the bad condition score and the value of the term based on the bad condition person discrimination model, and to calculate the probability of the bad condition based on the function y and the bad condition score. Further, the health management server 200C outputs the calculated value as the bad condition information and stores the bad condition information in the bad condition information database 250 in step S3302.

Because a process in step S3303 shown in FIG. 33 is similar to the process in step S1003 shown in FIG. 10, its description will be omitted.

Next, a process of the model generation unit 297 according to the fourth embodiment will be described with reference to FIG. 34. FIG. 34 is a flowchart for describing the process of the model generation unit 297 according to the fourth embodiment.

Because processes according to the bad condition person discrimination model generation unit 261A of the model generation unit 297 in step S3401 and step S3402 shown in FIG. 34 are similar to the process in step S1101 and step S1102 shown in FIG. 11, their descriptions will be omitted.

Subsequently, the model generation unit 297 causes the bad condition person discrimination model generation unit 261A to generate the bad condition person discrimination model in order to store the bad condition person discrimination model in the model storage unit 245 in step S3403. Subsequently, the model generation unit 297 causes the function model generation unit 262B to generate the function y in order to store the function y in the model storage unit 245 in step S3404. Then, the process ends. Because a process of the function model generation unit 262B according to the fourth embodiment in step S3404 shown in FIG. 34 is similar to the process shown in FIG. 29, its description will be omitted.

As described above, the fourth embodiment generates the function y when generating the bad condition person discrimination model. Thus, the fourth embodiment may output the bad condition score and the probability of the bad condition at the same timing when the bad condition information output unit 298 outputs the bad condition information.

Further, the present invention is not limited to these embodiments, but various variations and modifications may be made without departing from the scope of the present invention.

The present application is based on and claims the benefit of priority of Japanese Priority Application No. 2015-17624 filed on Jan. 30, 2015 and Japanese Priority Application No. 2015-240191 filed on Dec. 9, 2015 with the Japanese Patent Office, the entire contents of which are hereby incorporated by reference.

## Claims

1. An information processing apparatus for supporting extraction, from a plurality of managed persons, of one or more persons who are subject to an interview by a manager comprising:
an index value calculation unit configured to calculate, for each of the plurality of managed persons, an index value which is a criterion of determination whether a managed person is subject to the interview with reference to a work information database which stores work information and a health examination database which stores health examination information, the work information indicating a workplace attendance record of each of the plurality of the managed persons, the health examination information indicating a result of a health examination of each of the plurality of the managed persons;
a screen data generation unit configured to generate screen data for displaying a list screen, the list screen displaying the plurality of managed persons arranged in accordance with the index value; and
an output unit configured to output the screen data.

2. The information processing apparatus as claimed in claim 1, wherein the screen data generation unit is configured to generate the screen data for displaying, in the list screen, a selection field for selecting one or more managed persons from the plurality of managed persons arranged in accordance with the index value.

3. The information processing apparatus as claimed in claim 1 or 2,
wherein the index value indicates a probability of leave from work to be taken by the managed person, and
wherein the screen data generation unit is configured to generate the screen data for displaying, in the list screen, the plurality of managed persons in descending order of the probability.

4. The information processing apparatus as claimed in claim 1 or 2,
wherein the index value indicates a probability of leave from work to be taken by the managed person, and
wherein the screen data generation unit is configured to generate the screen data for displaying the plurality of managed persons in ascending order of the probability.

5. The information processing apparatus as claimed in any one of claim 1 to 4, further comprising:
a bad condition person discrimination model learning unit configured to generate a bad condition person discrimination model with reference to the work information database and the health examination database, the bad condition person discrimination model outputting a bad condition score used for calculating the index value for each of the plurality of managed persons;
a bad condition information generation unit configured to generate, for each of the plurality of managed persons, bad condition information which includes the bad condition score and the index value, the bad condition information generation unit storing identification information for identifying the managed person and the bad condition information in a bad condition information database in association with each other.

6. The information processing apparatus as claimed in claim 5, wherein the index value calculation unit is configured to calculate a percentage of managed persons each of who has a history of taking the leave from work among managed persons each of who has a bad condition score which matches a bad condition score of a managed person who is a calculation target of the index value with reference to the work information database and the health examination database.

7. The information processing apparatus as claimed in claim 5, wherein the index value calculation unit is configured to calculate, with reference to the work information database and the health examination database, a percentage of managed persons each of who has a history of taking the leave from work among managed persons each of whose bad condition scores is within a predetermined range, the predetermined range including a bad condition score of a managed person who is a calculation target of the index value.

8. The information processing apparatus as claimed in any one of claim 5 to 7, further comprising:
a function generation unit configured to generate a function based on the bad condition score and the index value, the function indicating a relationship between the bad condition score and the index value,
wherein the index value calculation unit is configured to calculate the index value based on the bad condition score and the function.

9. The information processing apparatus as claimed in any one of claim 5 to 8, further comprising:
a bad condition score calculation unit configured to output a sum of multiplied values as the bad condition score based on the bad condition person discrimination model, each of the multiplied values being obtained, for each item included in the work information database and the health examination database, by multiplying a value of each item by a coefficient determined with respect to each item.

10. The information processing apparatus as claimed in claim 9, further comprising:
a bad condition symptom extraction unit configured to extract, from each item of the work information database and/or the health examination database, one or more items in which the multiplied value are equal to or greater than a predetermined threshold for each of the plurality of managed persons,
wherein the screen data generation unit is configured to generate the screen data for displaying the list screen, the list screen displaying the one or more items which are extracted and the managed person in association with each other.

11. The information processing apparatus as claimed in any one of claim 1 to 10, further comprising:
a reader database configured to store identification information of the manager and an occupation of the manager in association with each other; and
an output information selection unit configured to select both the work information and the health examination information of the managed persons or the work information of the managed persons in accordance with the occupation of the manager,
wherein the screen data generation unit is configured to generate the screen data for displaying information selected by the output information selection unit.

12. A non-transitory recording medium for causing an information processing apparatus to execute a process, the information apparatus being for supporting extraction, from a plurality of managed persons, of one or more persons who are subject to an interview by a manager, the process comprising:
a step of calculating, for each of the plurality of managed persons, an index value which is a criterion of determination whether a managed person is subject to the interview with reference to a work information database which stores work information and a health examination database which stores health examination information, the work information indicating a workplace attendance record of each of the plurality of the managed persons, the health examination information indicating a result of a health examination of each of the plurality of the managed persons;
a step of generating screen data for displaying a list screen, the list screen displaying the plurality of managed persons arranged in accordance with the index value; and
a step of outputting the screen data.
